# EUROPEAN PATENT APPLICATION

(11) **EP 4 431 488 A1**
(43) Date of publication of application: **18.09.2024**
(21) Application number: 22888860.8
(22) Date of filing: 23.06.2022
(51) Int. Cl.: C07C 213/08, C07C 213/10, C07C 215/52

(54) **PREPARATION METHOD FOR DOPAMINE HYDROCHLORIDE**

(30) Priority: 08.11.2021 CN 202111318024
(71) Applicant: Wuhan Wuyao Pharmaceutical Co., Ltd., Huangshi, Hubei 435229 (CN)
(72) Inventor: ZHAO, Taotao, Huangshi, Hubei 435229 (CN); WANG, Zhaopu, Huangshi, Hubei 435229 (CN); ZHU, Di, Huangshi, Hubei 435229 (CN); HONG, Jian, Huangshi, Hubei 435229 (CN); LI, Tianqing, Huangshi, Hubei 435229 (CN); ZHANG, Qi, Huangshi, Hubei 435229 (CN)
(74) Representative: Cabinet Laurent & Charras
(86) International application number: PCT/CN2022/100644
(87) International publication number: WO 2023/077824

(57) **Abstract**

The present invention relates to the technical field of drug synthesis, and relates to a preparation method for dopamine hydrochloride. Specifically, the preparation method for dopamine hydrochloride of the present invention comprises the following steps: dissolving norepinephrine substances (norepinephrine ketone or norepinephrine) or hydrochloride thereof in a solvent, then adding a catalyst and optionally hydrochloric acid, and performing catalytic hydrogenation reaction to obtain dopamine hydrochloride. The method is simple in process and high in operability, raw materials are cheap and readily available, product purity and yield are high, and the method is less in pollution emission, environment-friendly, and suitable for industrial production.

## Description

### CROSS-REFERENCE TO RELATED APPLICATION

The present disclosure claims the priority to and the benefits of Chinese patent application No. 202111318024.8 filed on November 8, 2021 and entitled "PREPARATION METHOD FOR DOPAMINE HYDROCHLORIDE", the disclosures of which are incorporated herein by reference in its entirety.

### TECHNICAL FIELD

The present disclosure pertains to the technical filed of drug synthesis, and relates to a preparation method for dopamine hydrochloride.

### BACKGROUND

Dopamine hydrochloride is chemically known as 4-(2-aminoethyl)benzene-1,2-diol hydrochloride or 3-hydroxytyramine hydrochloride. Dopamine hydrochloride is a dopamine receptor agonist suitable for use in shock syndrome caused by myocardial infarction, trauma, endotoxic sepsis, cardiac surgery, renal failure, congestive heart failure, etc., and shock unable to be corrected even after replenishing blood volume, in particular shock with oliguria and normal or low peripheral vascular resistance. Because this compound may increase cardiac output, it is also used for cardiac insufficiency against which digitalis and diuretics are ineffective.

Dopamine was synthesized for the first time in 1910 by George Barger and James Ewens at Wellcome Laboratory in London, UK. Later, also in the Wellcome Laboratory, Henry Dale discovered that, similar to epinephrine, it acted as a weak sympathomimetic drug. In 1952, Henry Dale named it dopamine.

At present, there are mainly the following several preparation methods for dopamine hydrochloride reported in literatures:

### Route 1: using homopiperonylamine as a starting material (Chinese Journal of Pharmaceuticals, 1977, 2, 21-22)

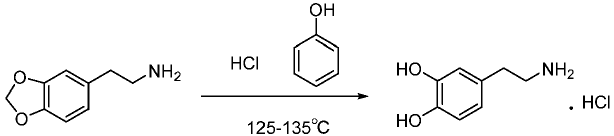

In this route, homopiperonylamine is used as a starting material and subjected to a one-step ring-opening reaction in the presence of hydrochloric acid and phenol to afford dopamine hydrochloride. This route requires simpler and fewer steps, but homopiperonylamine as the starting material is insufficient in supply and high in price. Besides, the reaction temperature is high, so there may be more by-products, and the one-step reaction makes itself difficult to control impurities.

### Route 2: using vanillin as a starting material (Journal of Shandong Institute of Light Industry, 1990, 4(3), 1-3)

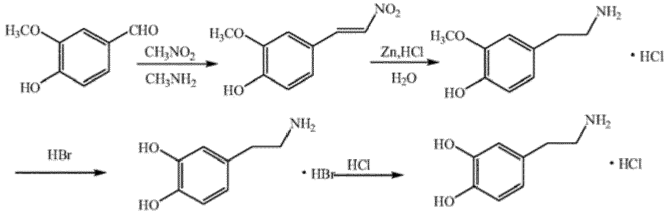

In this route, vanillin is used as a starting material and subjected to a four-step reaction comprising condensation, reduction, demethylation, and hydrochloric acid displacement to afford dopamine hydrochloride. As the starting material, vanillin is cheap and readily available, resulting in low cost. However, due to the presence of phenolic hydroxyl group in the structure, vanillin itself and the resulting intermediates with phenolic hydroxyl group(s) have poor stability and are prone to oxidation, leading to an increase in by-products. In addition, the reducing agent used in the reduction reaction as the second step is a system consisting of zinc powder and hydrochloric acid. Large amounts of zinc powder and hydrochloric acid are used, resulting in lots of waste with three forms.

### Route 3: using catechol as a starting material (S. G. Lee, et al., Enzyme and Microbial Technology, 25 (1999), 298-302)

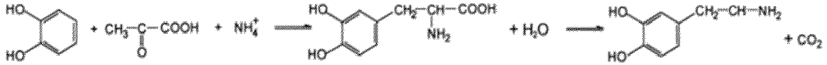

In this route, catechol is used as a raw material and reacts with pyruvic acid and ammonia to afford 3,4-dihydroxy-L-phenylalanine (L-DOPA), and L-DOPA is decarboxylated in the presence of L-DOPA decarboxylase (DDC; aromatic L-amino acid decarboxylase) to afford dopamine. This method is not suitable for industrial production because of harsh reaction conditions and high cost of decarboxylase.

### SUMMARY

### Problems to be solved

To solve the existing problem, the present disclosure is intended to provide a preparation method for dopamine hydrochloride. This method is featured in simple process, easy operability, cheap and readily available raw material, high purity and yield of product, and fewer pollution emissions and environmental friendliness, and is suitable for industrial production.

### Solution to the problems

[1] A preparation method for dopamine hydrochloride, comprising:
   dissolving a norepinephrine substance or hydrochloride thereof in a solvent, then adding a catalyst and optional hydrochloric acid, and performing a catalytic hydrogenation reaction to afford the dopamine hydrochloride;
   the norepinephrine substance is noradrenalone or norepinephrine;
   preferably, the norepinephrine substance is noradrenalone.
[2] The preparation method according to [1], characterized in that
   the solvent is water, methanol, ethanol or any combination thereof at any ratio, preferably water;
   preferably, the weight ratio of the norepinephrine substance or hydrochloride thereof to the solvent is 1:2 to 1:10, preferably 1:4 to 1:6.
[3] The preparation method according to [1], characterized in that
   the catalyst is at least one of platinum metals or compounds thereof loaded on at least one carrier, wherein the weight percentage of the at least one of platinum metals or compounds thereof relative to the catalyst is 0.5% to 10%;
   preferably, the catalyst is palladium on carbon, preferably palladium on carbon with a palladium content of 0.5 wt% to 10 wt%;
   preferably, the amount of the catalyst is 5 wt% to 50 wt%, preferably 20 wt% to 30 wt% of the norepinephrine substance or hydrochloride thereof.
[4] The preparation method according to [1], characterized in that
   in the presence of hydrochloric acid, the molar ratio of the norepinephrine substance or hydrochloride thereof to the hydrochloric acid is 1:1 to 1:5, preferably 1:2 to 1:3;
   preferably, the concentration of the hydrochloric acid is 1 mol/L.
[5] The preparation method according to [1], characterized in that
   the reaction temperature of the catalytic hydrogenation reaction is 30 to 150°C, preferably 70 to 100°C.
[6] The preparation method according to [1], characterized in that
   the reaction pressure of the catalytic hydrogenation reaction is 0.1 to 10 MPa, preferably 1 to 2 MPa.
[7] The preparation method according to [1], characterized in that
   the reaction duration of the catalytic hydrogenation reaction is 1 to 36 h, preferably 6 to 24 h.
[8] The preparation method according to [1], characterized in that
   the catalytic hydrogenation reaction is carried out in a tank reactor.
[9] The preparation method according to [1], characterized in that
   the preparation method further comprises performing post-processing; and
   the post-processing comprises:
      filtering a reaction solution obtained after completion of the catalytic hydrogenation reaction, concentrating a filtrate under reduced pressure, converting a concentrate obtained after reduced pressure concentration into slurry and filtering, and recrystallizing a filter cake and vacuum drying to afford the final product.
[10] The preparation method according to [9], characterized in that
   the pressure of the reduced pressure concentration is no less than 0.08 MPa, preferably 0.09 MPa;
   the temperature of the reduced pressure concentration is no lower than 50°C, preferably 65°C; and
   the reduced pressure concentration ends when the weight of the concentrate reaches 1.2 to 1.8 times, preferably 1.5 times the weight of the norepinephrine substance or hydrochloride thereof.
[11] The preparation method according to [9], characterized in that
   the conversion of the concentrate into slurry comprises:
   adding a dispersant into the concentrate; under stirring conditions, heating to 65 to 75°C and lasting for 1 to 3 h, and then cooling to 20 to 30°C and lasting for 0.5 to 1 h.
[12] The preparation method according to [11], characterized in that
   the dispersant is a liquid mixture of an alcohol and hydrochloric acid, wherein the weight ratio of the alcohol to the hydrochloric acid is 1:0.2 to 1:0.5;
   preferably, the dispersant is a liquid mixture of ethanol and hydrochloric acid, wherein the weight ratio of the ethanol to the hydrochloric acid is 1:0.3 to 1:0.4;
   more preferably, the concentration of the hydrochloric acid is 12 mol/L;
   preferably, the weight ratio of the concentrate to the dispersant is 1:1 to 1:1.5, preferably 1:3 to 1:1.4.
[13] The preparation method according to [9], characterized in that
   the recrystallization comprises:
   adding a good solvent to the filter cake; under stirring conditions, heating up until dissolved clarification, optionally adding activated carbon for decoloration and removing the activated carbon by filtration, and then adding an antisolvent and cooling to 5 to 10°C; filtering and washing a precipitate.
[14] The preparation method according to [13], characterized in that
   the good solvent is an alcohol or its aqueous solution;
   preferably, the good solvent is an aqueous ethanol solution, wherein the weight ratio of ethanol to water is 1:0.2 to 1:0.3;
   preferably, the weight ratio of the filter cake to the good solvent is 1:5 to 1:8.
[15] The preparation method according to [13], characterized in that
   the antisolvent is hydrochloric acid;
   preferably, the concentration of the hydrochloric acid is 12 mol/L;
   preferably, the weight ratio of the filter cake to the antisolvent is 1:1 to 1:2.
[16] The preparation method according to [9], characterized in that
   the temperature of the vacuum drying is 30 to 60°C, preferably 45 to 55°C;
   the duration of the vacuum drying is 1 to 5 h, preferably 2 to 3 h.

### Advantageous effects

The present disclosure provides a preparation method for dopamine hydrochloride. This method is featured in the simple process, easy operability, cheap and readily available raw material, high purity and yield of product, and fewer pollution emissions and environmental friendliness, and is suitable for industrial production.

### BRIEF DESCRIPTION OF THE DRAWINGS

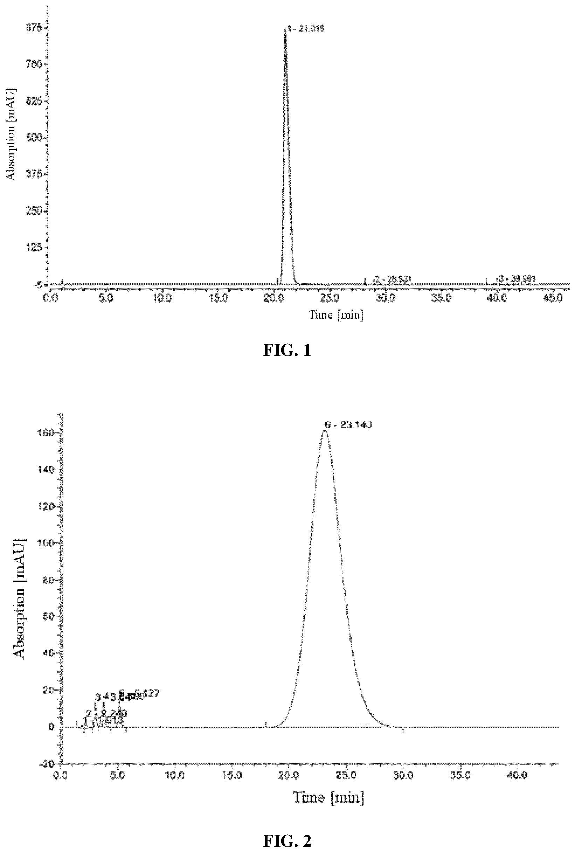

FIG. 1 is the liquid chromatogram of standard reference material of noradrenalone as a raw material.
FIG. 2 is the liquid chromatogram of norepinephrine as a raw material.

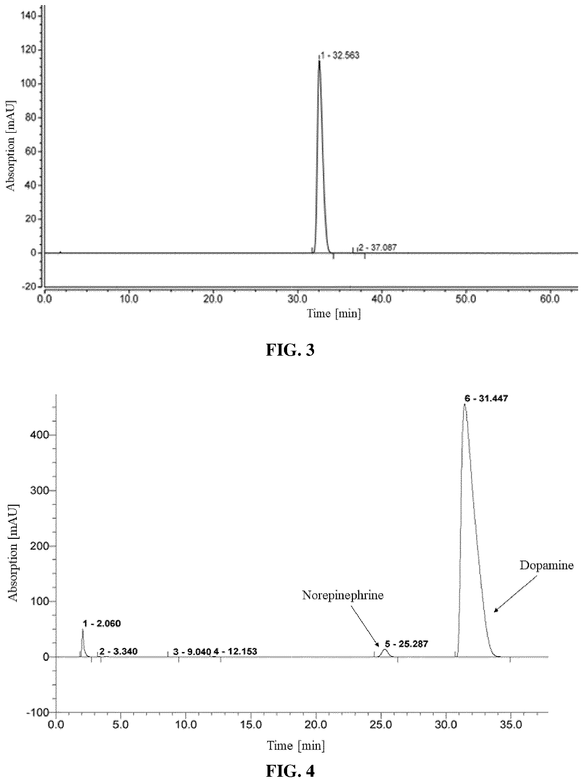

FIG. 3 is the liquid chromatogram of dopamine as a product.
FIG. 4 is the liquid chromatogram of norepinephrine and dopamine after completion of the reaction.

### DETAILED DESCRIPTION

In order to solve the problems existing in the prior art, the present disclosure provides a preparation method for dopamine hydrochloride.

Specifically, the preparation method may comprise: dissolving a reaction substrate in a solvent, then adding a catalyst and optional hydrochloric acid, and performing a catalytic hydrogenation reaction to afford dopamine hydrochloride.

In one embodiment of the present disclosure, the reaction substrate in the method may be noradrenalone represented by the structural formula below.

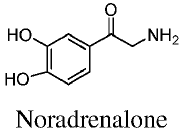

In one embodiment of the present disclosure, the reaction substrate in the method may be noradrenalone hydrochloride represented by the structural formula below.

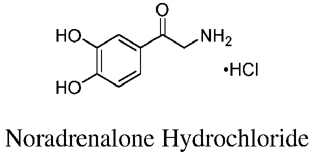

In one embodiment of the present disclosure, the reaction substrate in the method may be norepinephrine represented by the structural formula below.

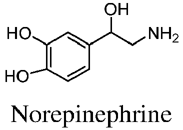

In one embodiment of the present disclosure, the reaction substrate in the method may be norepinephrine hydrochloride represented by the structural formula below.

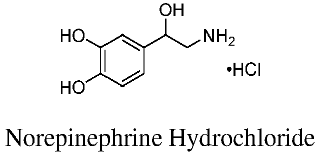

In one embodiment of the present disclosure, the solvent in the method can dissolve the reaction substrate. The solvent may be water, methanol, ethanol or any proportion of the combination thereof, *e.g*. water, methanol, ethanol, methanol/water, ethanol/water, methanol/ethanol, or methanol/ethanol/water.

In a preferred embodiment of the present disclosure, the solvent in the method may be water.

Water has a better dissolution effect on noradrenalone hydrochloride, while other organic solvents have relatively poor dissolution effects thereon.

In one embodiment of the present disclosure, the reaction substrate in the method has a certain dosage ratio to the solvent, and the weight ratio of the reaction substrate to the solvent (*e.g*. water) may be 1:2 to 1:10, for example, 1:2, 1:3, 1:4, 1:5, 1:6, 1:8, or 1:10.

In a preferred embodiment of the present disclosure, the weight ratio of the reaction substrate to the solvent (*e.g*. water) in the method may be 1:4 to 1:6, for example, 1:4, 1:4.5, 1:5, 1:5.5, or 1:6.

In case of using water as a solvent, when the weight ratio of noradrenalone (or hydrochloride thereof) to water is 1:2, dissolution needs to be performed at a higher temperature, and the temperature of dissolution is often higher than the temperature of reaction; when the weight ratio of noradrenalone (or hydrochloride thereof) to water is 1:10, a larger reaction equipment is required as a result of excessively large solvent volume, and the waste water will be increased, which is not conducive to practical production. Therefore, the appropriate weight ratio of the reaction substrate to the solvent is 1:4 to 1:6.

In a preferred embodiment of the present disclosure, the catalyst in the method may be at least one of platinum metals or compounds thereof loaded on at least one carrier, wherein the at least one of platinum metals or compounds thereof has a weight percentage of the catalyst in the range of 0.5% to 10%, for example, 0.5%, 1%, 1.5%, 2%, 5%, 8% or 10%.

Unless otherwise stated, the term "platinum metals" (also referred to as "platinum group metals") used herein refers to the noble metals of Group VIII in the Periodic Table of the Elements. Exemplary examples of the platinum metals include, but are not limited to, ruthenium (Ru), rhodium (Rh), palladium (Pd), osmium (Os), iridium (Ir), and platinum (Pt). Exemplary examples of the compounds of platinum metals include, but are not limited to, ruthenium trichloride (RuCl₃), palladium dichloride (PdCl₂), etc.

Unless otherwise stated, the term "carrier" (also referred to as "support") used herein refers to a component used to support a catalytically active component (*e.g*. the platinum metals or compounds thereof according to the present disclosure), so as to disperse the catalytically active component while enhancing the overall strength of the catalyst. Carrier is one of the components of the supported catalyst and is generally not catalytically active itself. Exemplary examples of the carrier include, but are not limited to, activated carbon, alumina, silica gel, diatomite, etc.

In a preferred embodiment of the present disclosure, the catalyst in the method may be palladium on carbon, and preferably palladium on carbon with a palladium content of 0.5 wt% to 10 wt%.

Compared with the other platinum metals, palladium is low-priced and palladium on carbon has a better conversion effect, so it is suitable to select palladium on carbon as the catalyst.

In one embodiment of the present disclosure, the reaction substrate in the method has a certain dosage ratio to the catalyst (*e.g*. 10 wt% of palladium on carbon). The amount of the catalyst may be 5 wt% to 50 wt% of the reaction substrate, for example, 5 wt%, 10 wt%, 15 wt%, 20 wt%, 30 wt%, 40 wt%, or 50 wt%.

In a preferred embodiment of the present disclosure, the amount of the catalyst (*e.g*. 10 wt% of palladium on carbon) in the method may be 20 wt% to 30 wt% of the reaction substrate.

The less the amount of the catalyst, the slower the catalytic progress; conversely, the more the amount of the catalyst, the higher the cost, and the potential safety hazards will also increase accordingly. Therefore, an appropriate amount of the catalyst (*e.g*. 10 wt% of palladium on carbon) is 20 wt% to 30 wt% of the reaction substrate.

In one embodiment of the present disclosure, the reaction substrate in the method may be noradrenalone hydrochloride or norepinephrine hydrochloride, and the catalytic hydrogenation reaction dispenses with extra addition of hydrochloric acid.

In one embodiment of the present disclosure, the reaction substrate in the method may be noradrenalone hydrochloride or norepinephrine hydrochloride, and the catalytic hydrogenation reaction requires extra addition of hydrochloric acid.

In one embodiment of the present disclosure, the reaction substrate in the method may be noradrenalone or norepinephrine, and the catalytic hydrogenation reaction requires extra addition of hydrochloric acid.

In case of requiring extra addition of hydrochloric acid, the molar ratio of the reaction substrate to the hydrochloric acid (measured by hydrogen chloride as the solute) in the method may be 1:1 to 1:5, for example, 1:1, 1:1.5, 1:2, 1:3, 1:4, or 1:5.

In one embodiment of the present disclosure, the molar ratio of the reaction substrate to the hydrochloric acid (measured by hydrogen chloride as the solute) in the method may be 1:2 to 1:3, for example, 1:2, 1:2.2, 1:2.4, 1:2.6, 1:2.8, or 1:3.

In a preferred embodiment of the present disclosure, the extraly added hydrochloric acid may be 1 mol/L hydrochloric acid.

If the concentration of hydrochloric acid is too high, it will cause great corrosion to the reaction equipment, which is not conducive to actual production; if the concentration of hydrochloric acid is too low, the reaction speed will be slower or the reaction is even difficult to proceed. Therefore, the appropriate concentration of the extraly added hydrochloric acid is 1 mol/L.

In one embodiment of the present disclosure, the reaction temperature of the catalytic hydrogenation reaction in the method may be 30 to 150°C.

In a preferred embodiment of the present disclosure, the reaction temperature of the catalytic hydrogenation reaction in the method may be 70 to 100°C.

As the reaction temperature rises, the content of impurities resulting from the catalytic hydrogenation reaction will also increase, such that both the yield and the purity of target product are affected. However, when the reaction temperature is lower, the reaction speed is slower or the reaction is even difficult to proceed. Therefore, the appropriate reaction temperature is 70 to 100°C.

In one embodiment of the present disclosure, the reaction pressure of the catalytic hydrogenation reaction in the method may be 0.1 to 10 MPa.

In a preferred embodiment of the present disclosure, the reaction pressure of the catalytic hydrogenation reaction in the method may be 1 to 2 MPa.

During the catalytic hydrogenation reaction, if the pressure of hydrogen is too low, the reaction rate will be slow, which is not conducive to practical production. However, if the pressure is too high, not only the amount of hydrogen required increases, but also the reaction equipment is required to be more pressure-resistant, which is not conducive to cost control. Therefore, the appropriate reaction pressure is 1 to 2 MPa.

In one embodiment of the present disclosure, the reaction duration of the catalytic hydrogenation reaction in the method may be 1 to 36 h.

In a preferred embodiment of the present disclosure, the reaction duration of the catalytic hydrogenation reaction in the method may be 6 to 24 h.

In a preferred embodiment of the present disclosure, the catalytic hydrogenation reaction in the method is carried out in a tank reactor (especially a hydrogenation reactor).

Asides from the above steps, the method may further comprise the step of performing post-processing. The post-processing step may comprise the sub-steps of: filtering a reaction solution obtained after completion of the catalytic hydrogenation reaction, concentrating a filtrate under reduced pressure, converting a concentrate obtained after reduced pressure concentration into slurry and filtering, and recrystallizing a filter cake and vacuum drying to afford the final product.

In one embodiment of the present disclosure, the sub-step of the reduced pressure concentration in the method may be carried out by a rotary evaporator.

In a preferred embodiment of the present disclosure, the pressure of the reduced pressure concentration may be no less than 0.08 MPa, preferably 0.09 MPa.

In a preferred embodiment of the present disclosure, the temperature of the reduced pressure concentration may be no lower than 50°C, preferably 65°C.

In one embodiment of the present disclosure, the reduced pressure concentration may end when the weight of the concentrate reaches 1.2 to 1.8 times the weight of the reaction substrate.

In a preferred embodiment of the present disclosure, the reduced pressure concentration may end when the weight of the concentrate reaches 1.5 times the weight of the reaction substrate.

In one embodiment of the present disclosure, the sub-step of the conversion of the concentrate into slurry in the method comprises: adding a dispersant into the concentrate; under stirring conditions, heating to 65 to 75°C and lasting for 1 to 3 h, and then cooling to 20 to 30°C and lasting for 0.5 to 1 h.

The dispersant in the sub-step of the conversion of the concentrate into slurry may be a liquid mixture of an alcohol and hydrochloric acid, wherein the weight ratio of the alcohol to the hydrochloric acid is 1:0.2 to 1:0.5, *e.g.* 1:0.2, 1:0.3, 1:0.4, or 1:0.5.

In one embodiment of the present disclosure, the dispersant in the sub-step of the conversion of the concentrate into slurry may be a liquid mixture of ethanol and hydrochloric acid (at a concentration of 12 mol/L), wherein the weight ratio of the ethanol to the hydrochloric acid is 1:0.3 to 1:0.4.

The concentrate has a certain dosage ratio to the dispersant in the sub-step of the conversion of the concentrate into slurry, in particular, the weight ratio of the concentrate to the dispersant may be 1:1 to 1:1.5, *e.g.* 1:1, 1:2, 1:3, 1:4, or 1:5.

In one embodiment of the present disclosure, the weight ratio of the concentrate to the dispersant may be 1:3 to 1:1.4, *e.g.* 1:3, 1:3.2, 1:3.4, 1:3.6, 1:3.8, or 1:4.

In one embodiment of the present disclosure, the sub-step of the recrystallization in the method comprises: adding a good solvent to the filter cake; under stirring conditions, heating up until dissolved clarification, optionally adding activated carbon for decoloration and removing the activated carbon by filtration, and then adding an antisolvent and cooling to 5 to 10°C; filtering and washing a precipitate.

The good solvent in the sub-step of the recrystallization may be an alcohol or its aqueous solution.

In one embodiment of the present disclosure, the good solvent in the sub-step of the recrystallization may be an aqueous ethanol solution, wherein the weight ratio of ethanol to water is 1:0.2 to 1:0.3, *e.g.* 1:0.22, 1:0.24, 1:0.26, 1:0.28, or 1:0.3.

The filter cake and the good solvent in the sub-step of the recrystallization are weighed at a certain ratio, in particular, the weight ratio of the filter cake to the good solvent (*e.g.* an aqueous ethanol solution) may be 1:5 to 1:8, *e.g.* 1:5, 1:6, 1:7, or 1:8.

In one embodiment of the present disclosure, the antisolvent in the sub-step of the recrystallization may be hydrochloric acid (at a concentration of 12 mol/L).

The filter cake has a certain dosage ratio to the antisolvent in the sub-step of the recrystallization, in particular, the weight ratio of the filter cake of the antisolvent (*e.g*. hydrochloric acid) is 1:1 to 1:2, *e.g.* 1:1, 1:1.2, 1:1.4, 1:1.6, 1:1.8, or 1:2.

In one embodiment of the present disclosure, the sub-step of the vacuum drying in the method may be carried out in a vacuum drying oven.

In a preferred embodiment of the present disclosure, the temperature of the vacuum drying may be 30 to 60°C, preferably 45 to 55°C.

In a preferred embodiment of the present disclosure, the duration of the vacuum drying may be 1 to 5 h, preferably 2 to 3 h.

The present disclosure will be further illustrated below with reference to specific examples. It shall be appreciated that the following examples are used only to explain or clarify the technical scheme of the present disclosure, rather than to restrict or limit the scope of the present disclosure in any form.

Unless otherwise stated, the reagents, materials, instruments, etc. used in the following embodiments may be obtained by conventional commercial means.

The dopamine of the present disclosure is subjected to HPLC analysis in accordance with General Chapters <0512> High-Performance Liquid Chromatography of the Chinese Pharmacopoeia (2020 edition) Volume IV*.*

### Chromatographic conditions:

stationary phase (filling agent): octadecylsilyl-bonded silica gel (ODS-18);
mobile phase: aqueous solution of sodium dodecyl sulfate (SDS) (0.005 M)-acetonitrile-glacial acetic acid-aqueous solution of ethylenediaminetetraacetic acid disodium salt (EDTA-2Na) (0.1 M) (700:300:10:2, v/v/v/v);
detection wavelength: 280 nm;
injection volume: 20 µl;
flow rate: 1.0 mL/min;
column temperature: 25°C.

### Example 1: Preparation of dopamine hydrochloride from noradrenalone hydrochloride (without extra addition of hydrochloric acid in the catalytic hydrogenation reaction)

Refined noradrenalone hydrochloride (2 g) was precisely weighed and placed in a hydrogenation reactor. Water (20 g, at a substrate/solvent weight ratio of 1:10) was added as a reaction solvent, and then Pd/C powder (0.4 g, in an amount of 20 wt% with respect to the substrate) with a palladium content of 10 wt% was added as a catalyst. The reactor was sealed and nitrogen was injected into the reactor to replace air. After three times of replacement, hydrogen was injected into the reactor to replace nitrogen until the pressure of hydrogen reached 2 MPa. The temperature-rising program of the reactor was set. Reaction was carried out at 100°C for 24 h. After completion of the reaction, the resultant was cooled and the hydrogen was released. After the replacement of nitrogen, reaction solution was taken out and filtered to remove palladium on carbon, thereby affording a colorless transparent liquid.

The resulting colorless transparent liquid was transferred to a single-neck flask and concentrated under reduced pressure at 60 to 65°C until the weight of concentrate was about 1.5 times the weight of noradrenalone hydrochloride. Then a mixed solution of anhydrous ethanol (in an amount of about 1.5 times the weight of noradrenalone hydrochloride) and hydrochloric acid (in an amount of about 0.59 times the weight of noradrenalone hydrochloride) was added to the single-neck flask. The system was placed in a water bath at 65 to 75°C and stirred for 2 h, then cooled to 20 to 22°C for heat preservation and stirred for 0.5 h, and subjected to suction filtration. Filter cake was washed twice with anhydrous ethanol, vacuum dried at 45 to 55°C for 3 h, and then collected to afford a crude product.

The above crude product was put into a three-neck flask, to which purified water (in an amount of about 1.05 times the weight of the crude product) and anhydrous ethanol (in an amount of about 4.70 times the weight of the crude product) were added. The system was placed in a water bath and heated until solution was clear. Thereafter, the solution was treated by adding thereto activated carbon (in an amount of about 0.01 times the weight of the crude product), stirred for 15 min, and subjected to suction filtration. Filtrate was transferred and placed in a water bath until solution was clear again. The solution was treated by adding thereto hydrochloric acid (in an amount of about 1.18 times the weight of the crude product), cooled to 5 to 10°C, stirred for 0.5 h, and subjected to suction filtration. Filter cake was washed twice with anhydrous ethanol, vacuum dried at 45 to 55°C for 3 h, and then collected to afford the final product.

After the HPLC analysis, it was shown that the purity could reach 99.84%.

### Example 2 to Example 13: Preparation of dopamine hydrochloride from noradrenalone hydrochloride (without extra addition of hydrochloric acid in the catalytic hydrogenation reaction)

Dopamine hydrochlorides of Examples 2 to 13 were prepared by referring to the preparation method of Example 1 and adjusting the process parameters accordingly. The process parameters and purity results in the Examples were as shown in Table 1.

**Table 1. Process Parameters in Examples 1 to 13**

| **Example** | **Substrate /g** | **Solvent** | **Substrate/Solvent Weight Ratio** | **Relative Amount of Catalyst /wt%** | **Temp. /°C** | **Pressure /MPa** | **Duration /h** | **Purity /%** | **Yield/%** |
|---|---|---|---|---|---|---|---|---|---|
| 1 | 2 | Water | 1:10 | 20 | 100 | 2 | 24 | 99.84 | 65.3 |
| 2 | 5 | Water | 1:4 | 20 | 100 | 2 | 24 | 97.87 | 64.7 |
| 3 | 6 | Water | 1:3 | 20 | 100 | 2 | 24 | 92.91 | 58.6 |
| 4 | 5 | Water | 1:6 | 20 | 120 | 2 | 24 | 96.65 | 62.8 |
| 5 | 5 | Water | 1:6 | 20 | 150 | 2 | 24 | 95.68 | 63.9 |
| 6 | 5 | Water | 1:6 | 20 | 100 | 1.5 | 24 | 97.38 | 62.3 |
| 7 | 5 | Water | 1:6 | 20 | 100 | 1 | 24 | 97.23 | 64.5 |
| 8 | 5 | Water | 1:6 | 20 | 100 | 0.5 | 24 | 96.91 | 63.6 |
| 9 | 5 | Water | 1:6 | 50 | 100 | 2 | 24 | 98.31 | 68.9 |
| 10 | 5 | Water | 1:6 | 30 | 100 | 2 | 24 | 97.98 | 63.4 |
| 11 | 5 | Water | 1:6 | 10 | 100 | 2 | 24 | 95.84 | 64.7 |
| 12 | 5 | Water | 1:4 | 20 | 100 | 2 | is | 92.32 | 61.7 |
| 13 | 5 | Water | 1:4 | 20 | 100 | 2 | 36 | 95.65 | 65.3 |

### Example 14: Preparation of dopamine hydrochloride from noradrenalone hydrochloride (with extra addition of hydrochloric acid in the catalytic hydrogenation reaction)

Refined noradrenalone hydrochloride (20 g) was precisely weighed and placed in a three-neck flask. Water (120 g, at a substrate/solvent weight ratio of 1:6) was added as a reaction solvent, heated at 65°C until dissolution, transferred to a hydrogenation reactor, and cooled to room temperature. To the reactor, hydrochloric acid (1 M, 13 g) was added, and then Pd/C powder (2 g, in an amount of 10 wt% with respect to the substrate) with a palladium content of 10 wt% as catalyst was added. The reactor was sealed and nitrogen was injected into the reactor to replace air. After three times of replacement, hydrogen was injected into the reactor to replace nitrogen until the pressure of hydrogen reached 1 MPa. The temperature-rising program of the reactor was set. Reaction was carried out at 70°C for 5 h. After completion of the reaction, the resultant was cooled and the hydrogen was released. After the replacement of nitrogen, reaction solution was taken out and filtered to remove palladium on carbon, thereby affording a colorless transparent liquid.

The resulting colorless transparent liquid was transferred to a single-neck flask and concentrated under reduced pressure at 60 to 70°C until the weight of concentrate was about 1.5 times the weight of noradrenalone hydrochloride. Then a mixed solution of anhydrous ethanol (in an amount of about 1.5 times the weight of noradrenalone hydrochloride) and hydrochloric acid (in an amount of about 0.59 times the weight of noradrenalone hydrochloride) was added to the single-neck flask. The system was placed in a water bath at 60 to 70°C and stirred for 2 h, then cooled to 10 to 25°C for heat preservation and stirred for 0.5 h, and subjected to suction filtration. Filter cake was washed twice with anhydrous ethanol, vacuum dried at 40 to 45°C for 3 h, and then collected to afford a crude product.

The above crude product was put into a three-neck flask, to which purified water (in an amount of about 1.05 times the weight of the crude product) and anhydrous ethanol (in an amount of about 4.70 times the weight of the crude product) were added. The system was placed in a water bath and heated until solution was clear. Thereafter, the solution was treated by adding thereto activated carbon (in an amount of about 0.01 times the weight of the crude product), stirred for 15 min, and subjected to suction filtration. Filtrate was transferred and placed in a water bath until solution was clear again. The solution was treated by adding thereto hydrochloric acid (in an amount of about 1.18 times the weight of the crude product), cooled to 5 to 10°C, stirred for 0.5 h, and subjected to suction filtration. Filter cake was washed twice with anhydrous ethanol, vacuum dried at 45 to 55°C for 3 h, and then collected to afford the final product.

After the HPLC analysis, it was shown that the purity could reach 99.89%, and the yield was 78.2%.

### Example 15 to Example 31: Preparation of dopamine hydrochloride from noradrenalone hydrochloride (with extra addition of hydrochloric acid in the catalytic hydrogenation reaction)

Dopamine hydrochlorides of Examples 15 to 31 were prepared by referring to the preparation method of Example 14 and adjusting the process parameters accordingly. The process parameters and purity results in the Examples were as shown in Table 2.

**Table 2. Process Parameters in Examples 14 to 31**

| **Example** | **Raw Material /g** | **Solvent** | **Substrate/ Solvent Weight Ratio** | **Relative Amount of Catalyst /wt%** | **Acid /g** | **Temp. /°C** | **Pressure /MPa** | **Duration /h** | **Purity /%** | **Yield /%** |
|---|---|---|---|---|---|---|---|---|---|---|
| 14 | 20 | Water | 1:6 | 10 | 13 (HCl) | 70 | 1 | 5 | 99.89 | 78.2 |
| 15 | 20 | Methanol | 1:10 | 10 | 13 (HCl) | 70 | 1 | 4.5 | 99.77 | 44.5 |
| 16 | 20 | Ethanol | 1:10 | 10 | 13 (HCl) | 70 | 1 | 4.5 | 99.16 | 32.8 |
| 17 | 20 | Water | 1:6 | 10 | 13 (HCl) | 100 | 1 | 3 | 99.87 | 69.5 |
| 18 | 20 | Water | 1:6 | 10 | 13 (HCl) | 85 | 1 | 4.5 | 99.62 | 77.4 |
| 19 | 20 | Water | 1:6 | 10 | 13 (HCl) | 45 | 1 | 8 | 99.17 | 51.2 |
| 20 | 20 | Water | 1:5 | 10 | 13 (HCl) | 70 | 1 | 5 | 99.89 | 75.9 |
| 21 | 20 | Water | 1:4 | 10 | 13 (HCl) | 70 | 1 | 5 | 99.71 | 74.1 |
| 22 | 20 | Water | 1:3 | 10 | 13 (HCl) | 70 | 1 | 5 | 99.85 | 66.4 |
| 23 | 20 | Water | 1:6 | 10 | 6.4 (H₂SO₄) | 70 | 1 | 3 | 99.92 | 41.8 |
| 24 | 20 | Water | 1:6 | 10 | 10.5 (HBr) | 70 | 1 | 4.5 | 99.46 | 49.7 |
| 25 | 20 | Water | 1:6 | 10 | 17.1 (HI) | 70 | 1 | 4.5 | 99.44 | 40.6 |
| 26 | 20 | Water | 1:6 | 10 | 13 (HCl) | 70 | 1.5 | 5 | 99.73 | 77.4 |
| 27 | 20 | Water | 1:6 | 10 | 13 (HCl) | 70 | 0.5 | 6 | 99.23 | 57.1 |
| 28 | 20 | Water | 1:6 | 10 | 13 (HCl) | 70 | 1 | 4.5 | 99.81 | 78.9 |
| 29 | 20 | Water | 1:6 | 10 | 13 (HCl) | 70 | 1 | 6 | 99.45 | 75.7 |
| 30 | 20 | Water | 1:6 | 15 | 13 (HCl) | 70 | 1 | 7 | 99.69 | 59.4 |
| 31 | 2000 | Water | 1:6 | 6 | 1300 (HCl) | 70 | 1 | 5.5 | 99.80 | 79.1 |

As can be seen from the results in Table 1 and Table 2, if the temperature of reaction is too high, the impurities will increase; and if the temperature is too low, the conversion rate of the raw material will be low, and it will be converted into an intermediate state, which affects the quality and yield of the product. The lower the amount (concentration) of the acid, the slower the reduction rate of the raw material, the more impurities will be produced, which affects the quality and yield of the product. The more the amount of the acid, the higher the requirements for the equipment, which increases the production cost. The lower the pressure, the harder it is to convert the raw material, which affects the yield; and the higher the pressure, the higher the requirements for the equipment, the greater the safety risk though without any significant influence on the reaction. The less the amount of the catalyst (Pd/C), the lower the efficiency of the hydrogenation reduction; and if the amount is higher, the reaction cost will be greatly increased.

## Claims

1. A preparation method for dopamine hydrochloride, comprising:
dissolving a norepinephrine substance or hydrochloride thereof in a solvent, then adding a catalyst and optional hydrochloric acid, and performing a catalytic hydrogenation reaction to afford the dopamine hydrochloride;
the norepinephrine substance is noradrenalone or norepinephrine;
preferably, the norepinephrine substance is noradrenalone.

2. The preparation method according to claim 1, **characterized in that**
the solvent is water, methanol, ethanol or any combination thereof at any ratio, preferably water;
preferably, the weight ratio of the norepinephrine substance or hydrochloride thereof to the solvent is 1:2 to 1:10, preferably 1:4 to 1:6.

3. The preparation method according to claim 1, **characterized in that**
the catalyst is at least one of platinum metals or compounds thereof loaded on at least one carrier, where the weight percentage of the at least one of platinum metals or compounds thereof relative to the catalyst is 0.5% to 10%;
preferably, the catalyst is palladium on carbon, preferably palladium on carbon with a palladium content of 0.5 wt% to 10 wt%;
preferably, the amount of the catalyst is 5 wt% to 50 wt%, preferably 20 wt% to 30 wt% of the norepinephrine substance or hydrochloride thereof.

4. The preparation method according to claim 1, **characterized in that**
in the presence of hydrochloric acid, the molar ratio of the norepinephrine substance or hydrochloride thereof to the hydrochloric acid is 1:1 to 1:5, preferably 1:2 to 1:3;
preferably, the concentration of the hydrochloric acid is 1 mol/L.

5. The preparation method according to claim 1, **characterized in that**
the reaction temperature of the catalytic hydrogenation reaction is 30 to 150°C, preferably 70 to 100°C.

6. The preparation method according to claim 1, **characterized in that**
the reaction pressure of the catalytic hydrogenation reaction is 0.1 to 10 MPa, preferably 1 to 2 MPa.

7. The preparation method according to claim 1, **characterized in that**
the reaction duration of the catalytic hydrogenation reaction is 1 to 36 h, preferably 6 to 24 h.

8. The preparation method according to claim 1, **characterized in that**
the catalytic hydrogenation reaction is carried out in a tank reactor.

9. The preparation method according to claim 1, **characterized in that**
the preparation method further comprises performing post-processing; and
the post-processing comprises:
filtering a reaction solution obtained after the completion of catalytic hydrogenation reaction, concentrating a filtrate under reduced pressure, converting a concentrate obtained after reduced pressure concentration into slurry and filtering, and recrystallizing a filter cake and vacuum drying to afford the final product.

10. The preparation method according to claim 9, **characterized in that**
the pressure of the reduced pressure concentration is no less than 0.08 MPa, preferably 0.09 MPa;
the temperature of the reduced pressure concentration is no lower than 50°C, preferably 65°C;
the reduced pressure concentration ends when the weight of the concentrate reaches 1.2 to 1.8 times, preferably 1.5 times the weight of the norepinephrine substance or hydrochloride thereof.

11. The preparation method according to claim 9, **characterized in that**
the conversion of the concentrate into slurry comprises:
adding a dispersant into the concentrate; under stirring conditions, heating to 65 to 75°C and lasting for 1 to 3 h, and then cooling to 20 to 30°C and lasting for 0.5 to 1 h.

12. The preparation method according to claim 11, **characterized in that**
the dispersant is a liquid mixture of an alcohol and hydrochloric acid, wherein the weight ratio of the alcohol to the hydrochloric acid is 1:0.2 to 1:0.5;
preferably, the dispersant is a liquid mixture of ethanol and hydrochloric acid, wherein the weight ratio of the ethanol to the hydrochloric acid is 1:0.3 to 1:0.4;
more preferably, the concentration of the hydrochloric acid is 12 mol/L;
preferably, the weight ratio of the concentrate to the dispersant is 1:1 to 1:1.5, preferably 1:3 to 1:1.4.

13. The preparation method according to claim 9, **characterized in that**
the recrystallization comprises:
adding a good solvent to the filter cake; under stirring conditions, heating up until dissolved clarification, optionally adding activated carbon for decoloration and removing the activated carbon by filtration, and then adding an antisolvent and cooling to 5 to 10°C; filtering and washing a precipitate.

14. The preparation method according to claim 13, **characterized in that**
the good solvent is an alcohol or its aqueous solution;
preferably, the good solvent is an aqueous ethanol solution, wherein the weight ratio of ethanol to water is 1:0.2 to 1:0.3;
preferably, the weight ratio of the filter cake to the good solvent is 1:5 to 1:8.

15. The preparation method according to claim 13, **characterized in that**
the antisolvent is hydrochloric acid;
preferably, the concentration of the hydrochloric acid is 12 mol/L;
preferably, the weight ratio of the filter cake to the antisolvent is 1:1 to 1:2.

16. The preparation method according to claim 9, **characterized in that**
the temperature of the vacuum drying is 30 to 60°C, preferably 45 to 55°C;
the duration of the vacuum drying is 1 to 5 h, preferably 2 to 3 h.
